# EUROPEAN PATENT APPLICATION

(11) **EP 3 366 314 A1**
(43) Date of publication of application: **29.08.2018**
(21) Application number: 15844007.3
(22) Date of filing: 03.09.2015
(51) Int. Cl.: A61L 2/10, A61L 9/20, A61N 5/06, C02F 1/32, F24F 7/00

(54) **STERILIZING DEVICE FOR PRESSURIZED FLUID**

(30) Priority: 24.09.2014 JP 2014193218; 16.04.2015 JP 2015084446
(71) Applicant: Tokuyama Corporation, Shunan-shi, Yamaguchi 745-8648 (JP)
(72) Inventor: MATSUI, Shingo, Shunan-shi Yamaguchi 745-8648 (JP); HORII, Yuriko, Shunan-shi Yamaguchi 745-8648 (JP); YAMAMOTO, Reo, Shunan-shi Yamaguchi 745-8648 (JP); HIRONAKA, Keiichiro, Shunan-shi Yamaguchi 745-8648 (JP); HAMA, Yasutaka, Shunan-shi Yamaguchi 745-8648 (JP)
(74) Representative: Markfort, Iris-Anne Lucie
(86) International application number: PCT/JP2015/075022
(87) International publication number: WO 2016/047410

(57) **Abstract**

[Object] To provide an ultraviolet sterilizing apparatus that is capable of efficiently performing ultraviolet sterilization on a large volume of gas or pressurized liquid in a short time, can be made compact, and can be stably and safely used for a long time.

[Solving Means] A pressurized fluid sterilizing apparatus including a pressure-resistant container or piping that provides pressurizing space, the pressurized fluid sterilizing apparatus sterilizing pressurized fluid such as pressurized gas and pressurized liquid in the container or piping by irradiating the pressurized fluid with ultraviolet rays, the pressurized fluid sterilizing apparatus being characterized in that the pressurized gas or pressurized liquid in the pressurizing space is irradiated with ultraviolet rays by placing an optical member for ultraviolet emission such as a diffusion lens and a light guide plate in the pressurizing space, transmitting ultraviolet rays emitted from an ultraviolet light source such as an ultraviolet light emitting diode by using a light transmission path such as an optical fiber to the optical member for ultraviolet emission, and emitting the ultraviolet rays.

## Description

### Technical Field

The present invention relates to a pressurized fluid sterilizing apparatus for performing ultraviolet sterilization on pressurized fluid. More specifically, the present invention relates to a pressurized fluid sterilizing apparatus for performing ultraviolet sterilization by irradiating pressurized fluid, which contains pressurized gas obtained by pressurizing gas, liquefied gas, or pressurized liquid obtained by pressurizing liquid, with ultraviolet rays.

### Background Art

Because the ultraviolet rays with the wavelength of 200 to 350 nm have effects of not only affecting the nucleic acid that is the protoplasm of bacteria to cause the bacteria to lose the proliferating ability but also destroying the protoplasm to kill the bacteria, it is possible to perform sterilization by irradiating gas with such ultraviolet rays. As a light source of such ultraviolet rays, a low-pressure mercury lamp (so-called sterilization lamp) that emits light with the wavelength of 253.7 nm (resonance line of mercury), which is generated by discharge of mercury vapor at low pressure (approximately 0.1 Pa), is generally used and the sterilization lamp is widely used in various fields. Further, in recent years, there are also increasing examples where an ultraviolet light emitting diode (hereinafter, referred to also as UV-LED) is used as a light source of ultraviolet rays for sterilization (see Patent Literature 1).

Incidentally, in the food industry field, pharmaceutical industry field, medical field, and the like, the inside of the room space, closed space separated by a partition, air curtain, or the like, positive pressure space, or negative pressure space needs to be in a highly cleaned state (e.g., aseptic state) in some cases. Then, in order to achieve this, it needs to supply filtered or sterilized air to the space, or filter or sterilize the air inside the space or the air discharged from the space. Also in such a case, ultraviolet sterilization is used in some cases. However, because the volume of air to be processed is large, an ultraviolet ramp that is capable of emitting strong ultraviolet rays is used as a light source in many cases and a UV-LED is rarely used (see Patent Literatures 2 to 6).

On the other hand, also an apparatus for sterilizing the pressurized air by placing an ultraviolet ramp in the pressurizing space has been known (see Patent Literature 7).

### Citation List

### Patent Literature

Patent Literature 1: Japanese Patent Application Laid-open No. 2014-100206
Patent Literature 2: Japanese Patent Application Laid-open No. 1995-198178
Patent Literature 3: Japanese Examined Patent Application Publication No. 1994-34941
Patent Literature 4: Utility Model Registration No. 103406
Patent Literature 5: Japanese Patent Application Laid-open No. 2008-178374
Patent Literature 6: Japanese Patent Application Laid-open No. 2011-254800
Patent Literature 7: Japanese Patent Application Laid-open No. 1998-249128

### Disclosure of Invention

### Technical Problem

In order to sterilize a large volume of air by irradiating the air with ultraviolet rays, it needs to irradiate the entire air to be sterilized with a predetermined volume or more of ultraviolet rays. Therefore, it is hard to avoid an increase in size of the sterilizing apparatus. However, because the volume of air can be significantly reduced by being pressurized, it is considered that the size of the sterilizing apparatus can be reduced by using an apparatus disclosed in Patent Literature 7 in which the pressurized air is irradiated with ultraviolet rays.

However, in the apparatus disclosed in Patent Literature 7, there is a limit to the size reduction of the apparatus because a tank (container 7f in Fig. 1 of Patent Literature 7) is separately provided in a pressurized air supply line and an ultraviolet sterilization lamp is placed in the tank. Furthermore, the object of the apparatus is to prevent such a problem that microbes are discharged when the apparatus is activated again after the apparatus is stopped for a long time from occurring. Therefore, the tank needs to be placed on the discharge piping on the most downstream of the pressurized air supply line, and there is a significant limit to the installation conditions of the sterilizing apparatus itself due to the necessity. Furthermore, there is such a problem that mercury is discharged to the outside when the apparatus starts activating or in the case where the ultraviolet sterilization lamp is damaged by the shock due to a rapid change in pressure or flow rate along with the breakdown of a pressurization system, the damage of the piping, or the like.

### Solution to Problem

The present invention is a new pressurized fluid sterilizing apparatus that solves the above-mentioned object and sterilizes pressurized fluid such as pressurized gas or pressurized liquid (including liquefied gas) by irradiating the pressurized fluid with ultraviolet rays, the pressurized fluid sterilizing apparatus including: a pressure-resistant container or piping that provides pressurizing space; an ultraviolet irradiation device; and an optical member for ultraviolet emission.

The ultraviolet irradiation device includes an ultraviolet light source and a light transmission mechanism. The ultraviolet light source is placed outside the pressurizing space. The light transmission mechanism includes a light incident port, a light emission port, and a light transmission path, ultraviolet rays emitted from the ultraviolet light source entering the light incident port, the light transmission path transmitting the ultraviolet rays that enters the light incident port to the light emission port.

The optical member for ultraviolet emission is placed in the pressurizing space and optically connected to the light emission port. The optical member for ultraviolet emission is configured to emit the ultraviolet rays to pressurized fluid in the pressurizing space.

In the pressurized fluid sterilizing apparatus according to an embodiment of the present invention, the optical member for ultraviolet emission may be an optical fiber collimator, a lens diffusion plate, a diffusion lens, or a light guide plate. Further, a shock absorbing mechanism such as a buffer tank, a flow rate regulator, an accumulator, an air cylinder, and a pressure regulating bypass line may be placed upstream a part in the pressurizing space where the optical member for ultraviolet emission is placed. Further, an inner surface of the container or piping to which ultraviolet rays are applied may be formed of an ultraviolet reflecting material.

### Advantageous Effects of Invention

According to the pressurized fluid sterilizing apparatus of the present invention, it is possible to efficiently sterilize a large volume of gas such as air in a short time as compared with the existing sterilizing apparatus in which the gas is sterilized at normal pressure. Furthermore, because a light source such as an ultraviolet lamp is not placed in the pressurizing space, it is possible to make the apparatus very compact. Therefore, it is possible to place the apparatus also in facilities where the installation location has not been secured. Furthermore, because the ultraviolet lamp is not damaged in the pressurizing space, it is possible to stably and safely use the apparatus for a long time.

Further, there is substantially no spatial limitation in the installation location when placing the ultraviolet emission port in the pressurizing space, and the ultraviolet emission port can be placed also in narrow space, e.g., inside a piping. Therefore, it is possible to sterilize not only the gas at an arbitrary location on the pressurizing line but also the liquid in a pressurized state (pressurized liquid) transferred in a narrow piping. Furthermore, in the case where a filter or the like is placed in the pressurizing space, for example, it is also possible to prevent microbes from reproducing by irradiating the filter with ultraviolet rays.

### Brief Description of Drawings

[Fig. 1] Fig. 1 is a schematic diagram showing the vicinity of an installation part of an optical member for ultraviolet emission in an ultraviolet sterilizing apparatus according to a typical embodiment of the present invention.
[Fig. 2] Fig. 2 is a schematic diagram showing the vicinity of an installation part of an optical member for ultraviolet emission in an ultraviolet sterilizing apparatus according to another typical embodiment of the present invention.
[Fig. 3] Fig. 3 is a schematic diagram showing the vicinity of an installation part of an optical member for ultraviolet emission in an ultraviolet sterilizing apparatus according to still another typical embodiment of the present invention.
[Fig. 4] Fig. 4 is a schematic diagram showing the vicinity of an installation part of an optical member for ultraviolet emission in an ultraviolet sterilizing apparatus in which a pressure regulating bypass line is provided as a shock absorbing mechanism.
[Fig. 5] Fig. 5 is a schematic diagram showing the vicinity of an installation part of an optical member for ultraviolet emission in an ultraviolet sterilizing apparatus according to still another typical embodiment of the present invention. Mode(s) for Carrying Out the Invention

A pressurized fluid sterilizing apparatus according to an embodiment of the present invention is a pressurized fluid sterilizing apparatus including a pressure-resistant container or piping that provides pressurizing space, the pressurized fluid sterilizing apparatus sterilizing pressurized liquid such as pressurized gas and liquefied gas in the container or piping by irradiating the pressurized liquid with ultraviolet rays, the pressurized fluid sterilizing apparatus further including: "an ultraviolet irradiation device" including an ultraviolet light source and a light transmission mechanism including a light incident port, a light transmission path, and a light emission port; and "an optical member for ultraviolet emission" placed in the pressurizing space, the pressurized fluid sterilizing apparatus being characterized in that pressurized liquid such as pressurized gas and liquefied gas in the pressurizing space is irradiated with ultraviolet rays by placing the ultraviolet light source outside the pressurizing space, optically connecting the light emission port to the optical member for ultraviolet emission, transmitting ultraviolet rays emitted from the light source to the optical member for ultraviolet emission, and emitting the ultraviolet rays.

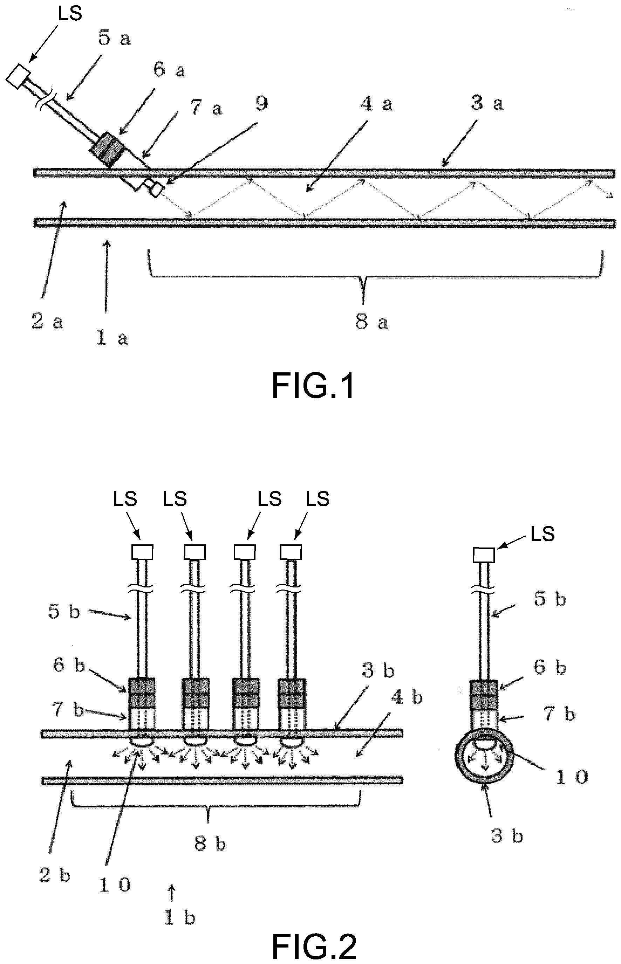

Note that the pressurizing space represents space where gas or liquid is under pressure higher than normal pressure (atmospheric pressure) or where gas is compressed and a part of the gas is liquefied. Examples of the pressurizing space include the following:
(1) pressurizing space (space in an air compressor, compressed air tank, air dryer, various filters, and piping connecting them) in a pneumatic line (compressible fluid circuit that may have a branch or confluence) for supplying compressed air, such as a flow system shown in Figs. 1 to 5 of Patent Literature 7 and a flow system shown in Figs. 1 and 2 of Japanese Unexamined Utility Model Application Publication No. 1994-74145;
(2) pressurizing space (see, for example, Fig. 1 of Japanese Patent Application Laid-open No. 1997-75459, Fig. 1 of Japanese Patent Application Laid-open No. 1998-15070, and a conceptual diagram of http://www.megacare.co.jp/feature/oxymed.html) in a medical gas supply system using a stationary liquefied gas supply apparatus or high-pressure gas manifold as a pressurized gas supply means instead of an air compressor;
(3) pressurizing space in a beer server shown in Fig. 1 of Japanese Patent Application Laid-open No. 2003-267493 or gas supply system for foaming milk using pressurized gas shown in Fig. 1 of Japanese Patent Application Laid-open No. 2014-502903;
   pressurizing space in a pressurized gas introduction apparatus shown in Fig. 1 of Japanese Patent Application Laid-open No. 2004-42648, breathing gas supply system shown in Fig. 2 of Japanese Unexamined Patent Application Publication No. 2005-503953, or gas supply system such as filtration facilities shown in Fig. 1 of Japanese Patent Application Laid-open No. 2011-110457; and
(4) space in a water piping.

Examples of the pressure-resistant container or piping that provides the pressurizing space include a compressor, a gas canister, a gas tank, a liquefied gas tank, a dryer, a gas cylinder, various line filters, an accumulator, a buffer tank, a silencer, a gas mixer, a temperature regulator, a humidity regulator, a pressure regulator, various valves, a metal piping, a pressure-resistant resin piping, a pressure-resistant hose, a coupler, various joints, a pressure gauge, and a flowmeter.

The gas to be pressurized or liquefied and sterilized is not particularly limited. However, for example, air, a carbonic acid gas, a nitrogen gas, a helium gas, an argon gas, an oxygen gas, nitrous oxide, a sterilization gas (mixed gas of ethylene and a carbonic acid gas) can be favorably used. Further, examples of the pressurized liquid include water and various drinks transferred in a piping.

Note that although the pressure at the time when the gas or liquid is sterilized by ultraviolet irradiation is not particularly limited as long as it is higher than atmospheric pressure, the pressure is favorably high because a large volume of gas can be sterilized in narrower space in the case where the gas is pressurized. From a viewpoint of the apparatus cost and application, the favorable pressure is normally within the range of not less than 0.2 MPa and not more than 10 MPa. More favorably, it is within the range of not less than 0.2 MPa and not more than 5 MPa, particularly, not less than 0.2 MPa and not more than 2 MPa. Further, in the case where the liquid is pressurized, the pressure is favorably low as long as the liquid can be transferred and the pressure is higher than atmospheric pressure, and is favorably within the range of more than 0.10133 MPa and not more than 1 MPa, particularly, not less than 0.102 MPa and not more than 0.800 MPa. Note that the pressure in the pressurizing space does not necessarily need to be constant in the entire space. Only the pressure in ultraviolet irradiation space (space where ultraviolet irradiation is performed in the pressurizing space) may be within the above-mentioned range by using a pressure-adjusting valve, a partition valve, or the like.

"The ultraviolet irradiation device" of the pressurized fluid sterilizing apparatus according to an embodiment of the present invention includes "the ultraviolet light source" and "the light transmission mechanism" including the light incident port, the light transmission path, and the light emission port. Then, the "ultraviolet light source" is placed outside the pressurizing space. In the ultraviolet irradiation device, ultraviolet rays emitted from the ultraviolet light source are taken in the light transmission path from the light incident port, and transmitted to the light emission port through the light transmission path. Then, the transmitted ultraviolet rays are emitted from the optical member for ultraviolet emission optically connected to the light emission port, and applied to pressurized gas, liquefied gas, or pressurized liquid in the pressurizing space.

As the light source, a high-pressure mercury lamp, low-pressure mercury lamp, xenon mercury lamp, xenon lamp, metal halide lamp, and ultraviolet light emitting diode (UV-LED) can be used. Among these light sources, it is more favorable to use an UV-LED that has a main light emission peak in a wavelength range of not less than 200 nm and less than 300 nm, particularly, not less than 220 nm and not more than 280 nm because the UV-LED has a high sterilization effect and is capable of utilizing the characteristics of LED.

Further, the light transmission path is not particularly limited as long as ultraviolet rays can be transmitted therethrough, and an optical fiber, an optical waveguide, a light guide plate, and the like can be used. Then, the light incident port is provided at one end portion of the light transmission path, and the light emission port is provided at the other end portion of the light transmission path. Further, the ultraviolet irradiation device used in the present invention may have a structure of a bundle fiber, an optical combiner, a coupler, an FGB, a collimator, and the like. As the ultraviolet irradiation device, a fiber bundle type UV-LED light source (manufactured by Fujikura Ltd., see http://www.fujikura.co.jp/f-news/1198834_4018.html) can be favorably used.

The optical member for ultraviolet emission placed in the pressurizing space in the pressurized fluid sterilizing apparatus according to an embodiment of the present invention is favorably an optical fiber collimator, lens diffusion plate, diffusion lens, or light guide plate, and particularly favorably, a lens diffusion plate, diffusion lens, or light guide plate because the irradiation area can be expanded.

Note that the optical fiber collimator is a member that makes light emitted from an optical fiber collimated light (parallel light), and a connector type one obtained by incorporating an aspheric lens into an optical fiber ferrule can be favorably used. The lens diffusion plate (Light Shaping Diffuser) is also called a diffusion film, diffusion filter, or diffusion sheet, and achieves uniform irradiation by diffusing and shaping light in a circular shape, elliptical shape, or the like by, for example, the effects of a minute lens randomly formed on the surface thereof. Further, as the diffusion lens, "Light Enhancer Cap" (registered trademark) manufactured by Enplas Corporation can be favorably used, for example. Furthermore, as the light guide plate, a surface light emission device disclosed in Japanese Patent Application Laid-open No 2006-237563 can be used, for example.

The installation location of the optical member for ultraviolet emission is not particularly limited as long as it is in the pressurizing space, and only needs to be determined from various pressure-resistant containers or piping from a view point of the purpose of irradiation and sterilization efficiency. Further, the size and shape of the optical member for ultraviolet emission, the number of optical member for ultraviolet emissions, the arrangement pattern in the case where many optical members for ultraviolet emission are placed, and the like only need to be appropriately determined depending on the size or shape of the space where the optical member for ultraviolet emission is placed. Note that the inner surface of the piping or pressure-resistant container in the ultraviolet irradiation space in the vicinity of a part where the optical member for ultraviolet emission is placed is favorably formed of a material having a high reflectance to ultraviolet rays, e.g., a platinum group metal such as Ru, Rh, Pd, Os, Ir, and Pt, Al, Ag, Ti, an alloy containing at least one of these metals, or magnesium oxide because the sterilization efficiency can be improved by the reflection of ultraviolet rays, and is particularly favorably formed of Al, a platinum group metal, an alloy containing a platinum group metal, or magnesium oxide because the reflectance is particularly high. Note that in the case where the surface is formed of these materials, the surface may be coated with an ultraviolet permeable material such as silicon dioxide.

The optical connection between the light emission port and the optical member for ultraviolet emission placed in the pressurizing space is not particularly limited as long as a manner in which airtightness in the pressurizing space is ensured is used, and the following manners:
1) a manner in which
   the optical member for ultraviolet emission has a main body provided inside the pressurizing space and a port part optically connectable to the light emission port, the port part being provided to be exposed outside the pressurizing space while maintaining the airtightness, and
   the port part of the optical member and the light emission port are optically connected to each other outside the pressurizing space; and
2) a manner in which
   a first optical fiber as a connecting element which is different from an optical fiber as the light transmission path is provided inside the pressurizing space by being airtightly inserted through an airtight adaptor or a pressure-resistant connector or a second optical fiber as a connecting element which is different from the optical fiber as the light transmission path is provided inside the pressurizing space to be airtightly connected to a third optical fiber as a connecting element which is different from the optical fiber as the light transmission path and provided outside the pressurizing space by using an airtight adaptor or a pressure-resistant connector, an inside portion of the first optical fiber as a connecting element or the second optical fiber as a connecting element and the optical member are optically connected to each other, and an outside portion of the first optical fiber as a connecting element or the third optical fiber as a connecting element and the light emission port are optically connected to each other
   can be used, for example.

In the case where the manner of the above-mentioned 1) is employed, it only needs to provide a flange part to the optical member for ultraviolet emission, make a hole in a piping or pressure-resistant container, through which a part (excluding the flange part) of the optical member for ultraviolet emission can be inserted, introduce a sealing structure such as a packing and an O-ring into the flange part, and directly fix or flange-fix it with a bolt and a nut, for example.

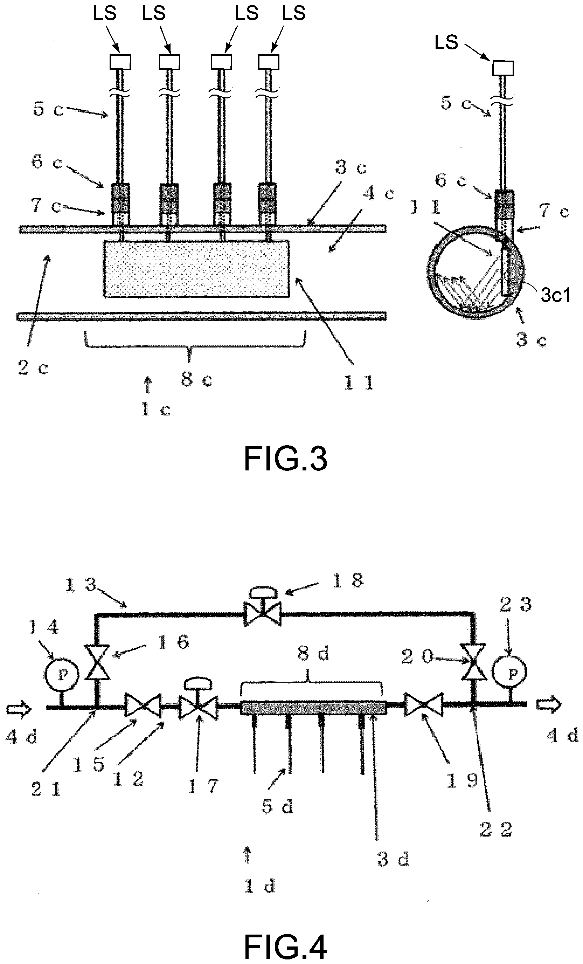

Further, examples of the airtight adaptor or pressure-resistant connector that can be employed in the manner of the above-mentioned 2) include airtight adaptors disclosed in Japanese Patent No. 3002966 and Japanese Patent No. 3335592, and a pressure-resistant optical connector described in Fig. 1 or Fig. 4 of Japanese Patent Application Laid-open No. 1994-250047. Note that in the case where a light waveguide or light guide plate is used as the light transmission path, it is possible to use such an adaptor or connector by using an optical fiber array or the like.

Fig. 1 to Fig. 3 respectively show a cross-sectional view of a part including ultraviolet irradiation spaces (8a, 8b, and 8c) of ultraviolet sterilizing apparatuses 1a, 1b, and 1c as a pressurized fluid sterilizing apparatus according to an embodiment of different aspects of the present invention. Further, Fig. 4 shows a partial piping diagram of an ultraviolet sterilizing apparatus 1d in which the ultraviolet irradiation space has a structure similar to that of the ultraviolet sterilizing apparatus 1c shown in Fig. 3 and a pressure regulating bypass line is provided as a shock absorbing mechanism.

Although an optical member for ultraviolet emission is placed in a piping, an optical fiber in pressurizing space and an optical fiber outside the pressurizing space are airtightly connected to each other by using a pressure-resistant connector, and the optical fiber in the pressurizing space is optically connected to the optical member for ultraviolet emission placed in the pressurizing space in any of these ultraviolet sterilizing apparatuses, the ultraviolet sterilizing apparatus of the present invention is not limited to such an aspect. For example, the installation location of the optical member for ultraviolet emission and the manner of optically connecting the light emission port and the optical member for ultraviolet emission to each other can be appropriately changed as necessary.

Hereinafter, the ultraviolet sterilizing apparatus of the present invention will be described in more detail with the ultraviolet sterilizing apparatuses of the present disclosure shown in these figures as examples.

Fig. 1 is a cross-sectional diagram showing the vicinity of an installation part of the optical member for ultraviolet emission in the ultraviolet sterilizing apparatus 1a, and the part includes the ultraviolet irradiation space 8a. In the part, a pressurizing space 2a is provided by a metal piping 3a, and a pressurized gas 4a supplied from a pressurized gas supply source (not shown) such as a compressor and a gas canister placed upstream through a pressure-resistant container such as a dryer and a line filter (any of which is not shown) placed as necessary flows or accumulates in the piping 3a. Further, a partition valve, pressure-adjusting valve, and the like (not shown) are placed downstream the part, and pressurized gas is released from the pressurizing space to the outside (e.g., atmosphere) by opening these valves.

In the piping 3a, an optical fiber collimator 9 is placed as the optical member for ultraviolet emission. The optical fiber collimator 9 is optically connected to an optical fiber that extends from a pressure-resistant connector 7a airtightly fixed through a hole obliquely provided to the piping 3a to the pressurizing space. The optical fiber that extends from the pressure-resistant connector 7a to the outside of the pressurizing space is optically connected to the light emission port of an optical fiber 5a that is the ultraviolet light transmission path of the light transmission mechanism in a coupler 6a.

The optical fiber 5a extends to the light incident port located at the other end portion. An ultraviolet light source LS is placed outside the piping 3a so as to face the light incident port of the optical fiber 5a. The ultraviolet light source LS is formed of an UV-LED that is capable of emitting ultraviolet rays having a main light emission peak in a wavelength range of not less than 200 nm and less than 300 nm with a high sterilization effect. The ultraviolet light source LS is optically connected to the light incident port so as to be able to emit the ultraviolet rays to the light incident port.

In the ultraviolet sterilizing apparatus 1a configured as described above, ultraviolet rays emitted from the ultraviolet light source LS are taken in from the light incident port, transmitted through the optical fiber 5a, and emitted from the optical fiber collimator 9 via the light emission port and the pressure-resistant connector 7a as parallel light. The emitted ultraviolet rays travel while being repeatedly reflected on the inner wall surface of the piping 3a formed of an ultraviolet reflecting material, and are applied to the pressurized gas 4a in the ultraviolet irradiation space 8a, thereby sterilizing the pressurized gas 4a. The emission direction of ultraviolet rays in the piping 3a may be a flow direction of the pressurized gas 4a or a direction opposite to the flow direction.

Note that the direction of the pressure-resistant connector 7a penetrating through the piping 3a is not limited to the direction oblique to the piping 3a, and may be vertical to the piping 3a. Further, as the optical member for ultraviolet emission, those capable of emitting and diffusing ultraviolet rays in the piping 3a, such a lens diffusion plate, a diffusion lens, and a light guide plate, may be employed. Accordingly, the irradiation area of ultraviolet rays can be expanded. Therefore, it is possible to efficiently sterilize the pressurized gas 4a.

Fig. 2 is a cross-sectional diagram showing the vicinity of an installation part of the optical member for ultraviolet emission in the ultraviolet sterilizing apparatus 1b, and the part includes the ultraviolet irradiation space 8b. In the part, a pressurizing space 2b is provided by a metal piping 3b, and a pressurized gas 4b supplied from a pressurized gas supply source (not shown) such as a compressor and a gas canister placed upstream through a pressure-resistant container such as a dryer and a line filter (any of which is not shown) placed as necessary flows or accumulates in the piping 3b. Further, a valve (not shown) is placed downstream the part, and pressurized gas is released from the pressurizing space to the outside by opening the valve.

In the piping 3b, a plurality of diffusion lenses 10 as the optical member for ultraviolet emission are placed. The plurality of diffusion lenses 10 are optically connected to a plurality of optical fibers that extend from a plurality of pressure-resistant connectors 7b airtightly fixed through a plurality of holes vertically provided to the piping 3b to the pressurizing space. The plurality of pressure-resistant connectors 7b are placed in a line in the axial direction of the piping 3b, and the respective optical fibers that extends from the plurality of pressure-resistant connectors 7b to the outside of the pressurizing space are optically connected to the light emission port of an optical fiber 5b that is the ultraviolet light transmission path of the light transmission mechanism in a coupler 6b.

The optical fibers 5b each extend to the light incident port located at the other end portion. A plurality of ultraviolet light sources LS are placed outside the piping 3b so as to face the respective light incident ports of the optical fibers 5b. The ultraviolet light sources LS are each formed of an UV-LED that is capable of emitting ultraviolet rays having a main light emission peak in a wavelength range of not less than 200 nm and less than 300 nm with a high sterilization effect. The ultraviolet light sources LS are each optically connected to the light incident port so as to be cable to emit the ultraviolet rays to the light incident port.

In the ultraviolet sterilizing apparatus 1b configured as described above, ultraviolet rays emitted from the respective ultraviolet light source LS are taken in from the light incident port, transmitted through the optical fiber 5b, and emitted from a diffusion lens 10 via the light emission port and the pressure-resistant connector 7a as diffusion light. The emitted ultraviolet rays are applied to the pressurized gas 4b in the ultraviolet irradiation space 8b while being repeatedly reflected on the inner wall surface of the piping 3a formed of an ultraviolet reflecting material, thereby sterilizing the pressurized gas 4b.

Note that the direction of the respective pressure-resistant connectors 7b penetrating through the piping 3b is not limited to the direction vertical to the piping 3b, and may be oblique to the piping 3a. Further, the arrangement direction of the pressure-resistant connectors 7b is not necessarily limited to a linear direction, and the pressure-resistant connectors 7b may be placed around the piping 3b in a staggered pattern or spiral pattern, for example. Further, the piping 3b does not necessarily need to be linear, and can be applied to a piping having a flexure. Furthermore, the ultraviolet light sources LS do not necessarily need to include a plurality of light sources placed corresponding to the respective optical fibers 5b, and may include a single light source common to the optical fibers 5b.

Fig. 3 is a cross-sectional diagram showing the vicinity of an installation part of the optical member for ultraviolet emission in the ultraviolet sterilizing apparatus 1c, and the part includes the ultraviolet irradiation space 8c. In the part, a pressurizing space 2c is provided by a metal piping 3c, and a pressurized gas 4c supplied from a pressurized gas supply source (not shown) such as a compressor and a gas canister placed upstream through a pressure-resistant container such as a dryer and a line filter (any of which is not shown) placed as necessary flows or accumulates in the piping 3c. Further, a valve (not shown) is placed downstream the part, and pressurized gas is released from the pressurizing space to the outside by opening the valve.

In the piping 3c, a light guide plate 11 as the optical member for ultraviolet emission is placed. The light guide plate 11 is formed in a rectangular shape having a longitudinal direction in parallel with the axial direction of the piping 3c. One main surface and the other main surface of the light guide plate 11 are respectively formed as a light emission surface and a supporting surface attached to a supporting portion 3c1 formed on a part of the inner surface of the piping 3c. A side surface (upper surface in Fig. 3) of the light guide plate 11 is optically connected to a plurality of optical fibers that extend from a plurality of pressure-resistant connectors 7c fixed to a plurality of holes vertically provided to the piping 3c to the pressurizing space. The plurality of pressure-resistant connectors 7c are placed in a line in the axial direction of the piping 3c, and the respective optical fibers that extend from the plurality of pressure-resistant connectors 7c to the outside of the pressurizing space are optically connected to the light emission port of an optical fiber 5c that is the ultraviolet light transmission path of the light transmission mechanism in a coupler 6c.

The optical fiber 5c extends to the light incident port located at the other end portion. A plurality of ultraviolet light sources LS are placed outside the piping 3c so as to face the respective light incident ports of the optical fibers 5c. The ultraviolet light sources LS are each formed of an UV-LED that is capable of emitting ultraviolet rays having a main light emission peak in a wavelength range of not less than 200 nm and less than 300 nm with a high sterilization effect. The ultraviolet light sources LS are each optically connected to the light incident port so as to emit ultraviolet rays to the light incident port.

In the ultraviolet sterilizing apparatus 1c configured as described above, ultraviolet rays emitted from the respective ultraviolet light source LS are taken in from the light incident port, transmitted through the optical fiber 5c, and emitted from the one main surface (light emission surface) of the light guide plate 11 via the light emission port and the pressure-resistant connector 7c as diffusion light. The emitted ultraviolet rays travel while being repeatedly reflected on the inner wall surface of the piping 3c formed of an ultraviolet reflecting material, and are applied to the pressurized gas 4c in the ultraviolet irradiation space 8c, thereby sterilizing the pressurized gas 4c.

In the ultraviolet sterilizing apparatus 1c configured as described above, because the light guide plate 11 is placed along the inner wall surface of the piping 3c, it is possible to perform ultraviolet sterilization on the pressurized gas 4c without disturbing the flow of the pressurized gas 4c even if the flow rate of the pressurized gas 4c in the piping 3c is relatively large. Note that in the case where the flow rate of the pressurized gas 4c in the piping 3c is relatively small or the pressurized gas 4c accumulates in the piping 3c, the light guide plate 11 may be placed along the central axis of the piping 3c. In this case, in the light guide plate 11, not only one main surface but also the other main surface opposite thereto may be configured as a light emission surface.

Fig. 4 is a partial piping diagram showing the ultraviolet sterilizing apparatus 1d in which ultraviolet irradiation space has a structure similar to that of the ultraviolet sterilizing apparatus 1c shown in Fig. 3 and a pressure regulating bypass line 13 is provided as a shock absorbing mechanism, and a main line 12 includes an ultraviolet irradiation space 8d. Further, a pressurized gas 4d is supplied from a pressurized gas supply source (not shown) such as a compressor and a gas canister placed upstream the piping part through a pressure-resistant container such as a dryer and a line filter (any of which is not shown) placed as necessary. Further, a valve (not shown) is placed downstream the part, and pressurized gas is released from the pressurizing space to the outside by opening the valve.

In the ultraviolet sterilizing apparatus 1d, the pressure regulating bypass line 13 is provided so as to branch from a branch point 21 located upstream the ultraviolet irradiation space 8d of the main line 12, bypass the ultraviolet irradiation space 8d, and be connected to the main line 12 at a confluence 22 located downstream the ultraviolet irradiation space 8d. Partition valves 15 and 16 are respectively provided right after the branch point 21 on the main line 12 and right after the branch point 21 on a bypass line 13. Partition valves 19 and 20 are respectively provided right before the confluence 22 on the main line 12 and right before the confluence 22 on the bypass line 13. With an opening/closing operation of these valves, it is possible to switch the flow path of the pressurized gas 4d. Further, a pressure gauge 14 for measuring the pressure on the upstream side of the branch point 21 and a pressure gauge 23 for measuring the pressure on the downstream side of the confluence 22 are respectively provided on the upstream side of the branch point 21 and the downstream side of the confluence 22. Furthermore, a flow rate regulating valve 17 and a flow rate regulating valve 18 are respectively provided downstream the partition valve 15 and upstream the ultraviolet irradiation space 8d on the main line 12 and between the partition valves 16 and 20 on the bypass line 13. Accordingly, it is possible to control the flow rate (flow velocity) of the pressurized gas 4d flowing through each line.

The bypass line 13 in the ultraviolet sterilizing apparatus 1d is placed to reduce the adverse effect on the apparatus by the shock due to a rapid change in pressure or flow rate when the apparatus starts activating (pressurized gas starts flowing), and the shock can be reduced by the following mechanism of action.

Specifically, the pressurized gas 4d is caused to flow first while the partition valves 15, 16, 19, and 20 and the flow rate regulating valves 17 and 18 are all closed at the time when the apparatus starts activating. After it is confirmed that the pressure upstream the branch point 21 reaches predetermined pressure, the partition valves 16 and 20 are sequentially opened. Then, by causing the pressurized gas 4d to flow to the bypass line 13 while gradually opening the flow rate regulating valve, the pressure downstream the confluence 22 is increased to equalize the pressure with the pressure on the upstream side of the branch point 21. After that, the flow rate regulating valve 18 and the partition valves 20 and 16 are closed, and the partition valve 15 is opened before the flow rate regulating valve 17 is gradually opened. Then, by opening the partition valve 19 after the pressure on the upstream side of the partition valve 19 is stable, pressurized gas is slowly introduced into the main line 12 between the partition valve 15 and the partition valve 19. Accordingly, it is possible to reduce the shock in the ultraviolet irradiation space 8d.

Although the ultraviolet sterilizing apparatus 1d shown in Fig. 4 is an example in which the pressure regulating bypass line 12 is provided as the shock absorbing mechanism and the branch point is provided upstream the ultraviolet irradiation space 8d, it is possible to achieve the similar reduction effects by placing a buffer tank, a flow rate regulator, an accumulator, an air cylinder, and the like upstream the ultraviolet irradiation space 8d.

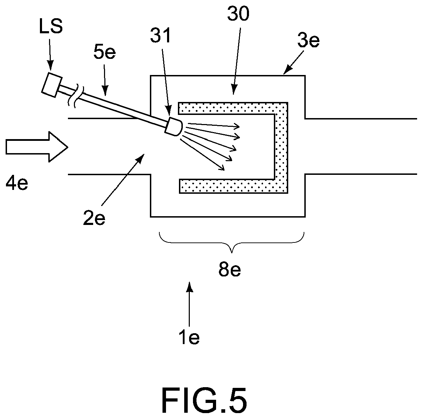

Fig. 5 is a cross-sectional diagram showing the vicinity of an installation part of the optical member for ultraviolet emission in an ultraviolet sterilizing apparatus 1e as a pressurized fluid sterilizing apparatus according to another embodiment, and the part includes an ultraviolet irradiation space 8e. A pressurizing space 2e is provided by a metal piping 3e in the part, a pressurized gas 4e supplied from a pressurized gas supply source (not shown) such as a compressor and a gas canister placed upstream is caused to flow through the piping 3e, a line filter 30 for filtering the pressurized gas 4e is placed in the ultraviolet irradiation space 8e of the piping 3e, a valve (not shown) is placed downstream the piping 3e, and pressurized gas is released from the pressurizing space to the outside by opening the valve.

In the piping 3e, for example, a diffusion lens 31 is placed as the optical member for ultraviolet emission. The diffusion lens 31 is optically connected to the light emission port of an optical fiber 5e that is the ultraviolet light transmission path via a pressure-resistant connector airtightly fixed through a hole obliquely provided to the piping 3e, a coupler, and the like, similarly to the first embodiment.

The optical fiber 5e extends to the light incident port located at the other end portion. An ultraviolet light source LS is placed outside the piping 3e so as to face the light incident port of the optical fiber 5e. The ultraviolet light source LS is formed of an UV-LED that is capable of emitting ultraviolet rays having a main light emission peak in a wavelength range of not less than 200 nm and less than 300 nm with a high sterilization effect. The ultraviolet light source LS is optically connected to the light incident port so as to be able to emit the ultraviolet rays to the light incident port.

In the ultraviolet sterilizing apparatus 1e configured as described above, ultraviolet rays emitted from the ultraviolet light source LS are taken in from the light incident port, transmitted through the optical fiber 5e, and emitted from the diffusion lens 31 via the light emission port as diffusion light. The emitted ultraviolet rays are applied to the pressurized gas 4a and the line filter 30 in the ultraviolet irradiation space 8e, thereby sterilizing the pressurized gas 4a and the line filter 30.

According to this embodiment, because not only the pressurized gas 4e but also the line filter 30 can be sterilized, it is possible to protect the line filter 30 from bacterial contamination and effectively prevent the pressurized gas 4e from being recontaminated (reattachment of bacteria) when passing through the line filter 30.

In the above-mentioned embodiment, although the ultraviolet sterilizing apparatus 1e configured as described above is provided to a part of the piping 3e, the ultraviolet sterilizing apparatus 1e may be configured as a single unit that can be attached to an outlet of a piping from which pressurized gas is released, for example. Specifically, because the outlet of the piping is easy to be in contact with external air, it is relatively easy to be contaminated by bacteria. In this regard, by attaching the ultraviolet sterilizing apparatus 1e including the line filter 30 and the like to the vicinity of the outlet of the piping, it is possible to stably release clean pressurized gas, which is not contaminated by bacteria, for a long time.

### Reference Signs List

1a, 1b, 1c, 1d, 1e ultraviolet sterilizing apparatus
2a, 2b, 2c, 2e pressurizing space
3a, 3b, 3c, 3d, 3e piping
4a, 4b, 4c, 4d, 4e pressurized gas
5a, 5b, 5c, 5d, 5e optical fiber
6a, 6b, 6c coupler
7a, 7b, 7c pressure-resistant connector
8a, 8b, 8c, 8d, 8e ultraviolet irradiation space
9 optical fiber collimator
10 diffusion lens
11 light guide plate
12 main line
13 pressure regulating bypass line
14 pressure gauge
15, 16 partition valve
17, 18 flow rate regulating valve
19, 20 partition valve
21 branch point
22 confluence
23 pressure gauge
30 line filter
31 diffusion lens

## Claims

1. A pressurized fluid sterilizing apparatus that sterilizes pressurized fluid by irradiating the pressurized fluid with ultraviolet rays, comprising:
a pressure-resistant container or piping that provides pressurizing space;
an ultraviolet irradiation device including an ultraviolet light source and a light transmission mechanism, the ultraviolet light source being placed outside the pressurizing space, the light transmission mechanism including a light incident port, a light emission port, and a light transmission path, ultraviolet rays emitted from the ultraviolet light source entering the light incident port, the light transmission path transmitting the ultraviolet rays that enters the light incident port to the light emission port; and
an optical member for ultraviolet emission, which is placed in the pressurizing space and optically connected to the light emission port, the optical member for ultraviolet emission being configured to emit the ultraviolet rays to pressurized fluid in the pressurizing space.

2. The pressurized fluid sterilizing apparatus according to claim 1, wherein
the pressurized fluid is pressurized gas or pressurized liquid.

3. The pressurized fluid sterilizing apparatus according to claim 1, wherein
the pressurized fluid is gas or liquefied gas under pressure of not less than 0.2 MPa and not more than 10 MPa or liquid under pressure of more than 0.10133 MPa and not more than 1 MPa.

4. The pressurized fluid sterilizing apparatus according to any one of claims 1 to 3, wherein
the optical member for ultraviolet emission is an optical fiber collimator, a lens diffusion plate, or a diffusion lens.

5. The pressurized fluid sterilizing apparatus according to any one of claims 1 to 3, wherein
the optical member for ultraviolet emission is a light guide plate, and the light output port of the light transmission mechanism is connected to a side surface of the light guide plate.

6. The pressurized fluid sterilizing apparatus according to any one of claims 1 to 5, further comprising
a shock absorbing mechanism placed upstream a part in the pressurizing space where the optical member for ultraviolet emission is placed

7. The pressurized fluid sterilizing apparatus according to any one of claims 1 to 6, wherein
an inner surface of the container or piping to which ultraviolet rays are applied is formed of an ultraviolet reflecting material.

8. The pressurized fluid sterilizing apparatus according to any one of claims 1 to 7, wherein
the container or piping includes a line filter, the pressurized fluid flowing through the line filter, and
the optical member for ultraviolet emission is configured to emit the ultraviolet rays to the line filter.
